# EUROPEAN PATENT APPLICATION

(11) **EP 4 549 470 A1**
(43) Date of publication of application: **07.05.2025**
(21) Application number: 23831311.8
(22) Date of filing: 23.06.2023
(51) Int. Cl.: C07K 19/00, A61K 9/08, A61K 45/00, A61K 47/42, A61P 43/00, C07K 14/00

(54) **PEPTIDE AND ASSEMBLY OR COMPOSITION CONTAINING SAID PEPTIDE**

(30) Priority: 29.06.2022 JP 2022104662
(71) Applicant: Mescue-Janusys Inc., Tokyo 103-0023 (JP)
(72) Inventor: HARADA Mitsunori, Tokyo 103-0023 (JP); YOSHIDA Chuya, Tokyo 103-0023 (JP)
(74) Representative: Müller-Boré & Partner Patentanwälte PartG mbB
(86) International application number: PCT/JP2023/023371
(87) International publication number: WO 2024/004869

(57) **Abstract**

The present invention provides a peptide applicable to an active targeting DDS. According to an embodiment of the present invention, there is provided a peptide or a derivative thereof or a salt thereof, including a hydrophobic region, and a hydrophilic region positioned on at least one end side of the hydrophobic region and having hydrophobicity lower than that of the hydrophobic region, including, at one end, a first target binding site capable of binding to a first target, and including, at the other end, a second target binding site capable of binding to a second target.

## Description

### Technical Field

The present invention relates to a peptide and an assembly or a composition including the peptide.

### Background Art

Peptides formed of several to several tens of amino acid residues have advantages, such as easy production and quality control at low cost, and less likelihood of antigenicity and easy permeation into a tissue, as compared to proteins such as antibodies. Meanwhile, peptides have an aspect of difficulty in direct application to pharmaceuticals due to properties, such as biological instability and aggregability.

In addition, hitherto, various drug delivery systems (DDSs) have been developed. For example, in Patent Literature 1, there is a disclosure that globular nano-microparticles formed by self-association of amphiphilic peptide molecules each including a hydrophilic part containing a positively-charged amino acid residue and a hydrophobic part covalently bonded to the hydrophilic part are used as a carrier preparation for a hydrophobic drug.

### Citation List

### Patent Literature

[PTL 1] JP 2017-525676 A

### Summary of Invention

### Technical Problem

In recent years, an active targeting DDS employing a target-directed molecule (ligand molecule) has been vigorously researched and developed, and target directivity of peptides, which have different properties from that of antibodies, has come to draw attention.

In view of the foregoing, a primary object of the present invention is to provide a peptide applicable to an active targeting DDS.

### Solution to Problem

According to one aspect of the present invention, there are provided a peptide of any one of the following items [1] to [8], a peptide assembly of any one of the following items [9] to [11], and a peptide composition of the following item [12] or [13].
[1] A peptide or a derivative thereof, or a salt thereof, including a hydrophobic region and a hydrophilic region positioned on at least one end side of the hydrophobic region having hydrophobicity lower than that of the hydrophobic region, including, at one end, a first target binding site capable of binding to a first target, and including, at the other end, a second target binding site capable of binding to a second target.
[2] The peptide or the derivative thereof, or the salt thereof according to Item [1], wherein the first target and the second target are different from each other.
[3] The peptide or the derivative thereof, or the salt thereof according to Item [1] or [2], wherein the number of amino acid residues for forming the peptide or the derivative thereof, or the salt thereof is from 10 to 40.
[4] The peptide or the derivative thereof, or the salt thereof according to any one of Items [1] to [3], wherein the peptide or the derivative thereof, or the salt thereof includes the hydrophilic regions on both end sides of the hydrophobic region.
[5] The peptide or the derivative thereof, or the salt thereof according to Item [4], wherein the number of amino acid residues for forming the hydrophobic region is from 2 to 15, and 60% or more of the amino acid residues are selected from a non-polar amino acid residue and a tyrosine residue.
[6] The peptide or the derivative thereof, or the salt thereof according to Item [4] or [5], wherein the numbers of amino acid residues for forming the two hydrophilic regions are each independently from 3 to 24.
[7] The peptide or the derivative thereof, or the salt thereof according to any one of Items [1] to [3], wherein the peptide or the derivative thereof, or the salt thereof includes the hydrophilic region at only one end of the hydrophobic region.
[8] The peptide or the derivative thereof, or the salt thereof according to any one of Items [1] to [7], wherein at least one of the first target binding site or the second target binding site has biological activity.
[9] A peptide assembly, including the peptide or the derivative thereof, or the salt thereof of any one of Items [1] to [8].
[10] The peptide assembly according to Item [9], further including a drug.
[11] The peptide assembly according to Item [9] or [10], wherein the peptide assembly satisfies at least one of the following item (i) or (ii):
   (i) binding affinity of the peptide assembly to the first target per the peptide or the derivative thereof, or the salt thereof is equal to or more than binding affinity of a non-self-associating peptide including the first target binding site to the first target; and
   (ii) binding affinity of the peptide assembly to the second target per the peptide or the derivative thereof, or the salt thereof is equal to or more than binding affinity of a non-self-associating peptide including the second target binding site to the second target.
[12] A peptide composition, including an aqueous medium and the peptide assembly of Item [9] or [11], wherein the peptide assembly is formed through association via an intermolecular interaction including a hydrophobic interaction between the hydrophobic regions of the peptides.
[13] The peptide composition according to Item [12], wherein the peptide assembly further includes a drug.

### Advantageous Effects of Invention

A peptide according to an embodiment of the present invention has a configuration in which the peptide includes a hydrophobic region having relatively high hydrophobicity and a hydrophilic region having relatively low hydrophobicity, and target binding sites each having affinity to a target are arranged on both ends thereof. The peptides each having such configuration can associate together through a hydrophobic interaction between the hydrophobic regions in an aqueous medium to form an assembly in which one end on a hydrophilic region side is arranged outside. The assembly is applicable to an active targeting DDS by, for example, further including a drug or employing a target binding site having biological activity.

### Brief Description of Drawings

FIG. **1(a)** and FIG. **1(b)** are schematic views for illustrating configurations of a peptide according to a first embodiment of the present invention and a peptide assembly formed of the peptide, respectively.
FIG. **2** is an illustration of results of helical wheel analysis for an example of a hydrophobic region.
FIG. **3** is a schematic view for illustrating relationships between target binding sites and a hydrophobic region or hydrophilic regions in the peptide according to the first embodiment of the present invention.
FIG. **4(a)** and FIG. **4(b)** are schematic views for illustrating configurations of a peptide according to a second embodiment of the present invention and a peptide assembly formed of the peptide, respectively.
FIG. **5** is a graph showing a relationship between a peptide concentration and a pyrene-derived fluorescence intensity.
FIG. **6** is a graph showing a relationship between a peptide concentration and an ANS-derived fluorescence intensity.
FIG. **7** is a graph showing results of evaluating blood circulation retention of a peptide.

### Description of Embodiments

Preferred embodiments of the present invention are described below. However, the present invention is not limited to these embodiments. In addition, the respective embodiments may be appropriately combined unless the combination is inappropriate in the context. Further, the expression "from ... to ..." indicating a numerical range as used herein includes the upper limit and lower limit values of the numerical range.

Herein, natural amino acids include alanine (A), leucine (L), arginine (R), lysine (K), asparagine (N), methionine (M), aspartic acid (D), phenylalanine (F), cysteine (C), proline (P), glutamine (Q), serine (S), glutamic acid (E), threonine (T), glycine (G), tryptophan (W), histidine (H), tyrosine (Y), isoleucine (I), and valine (V). In addition, non-natural amino acids include 2,3-diaminopropionic acid (DAP), 2,4-diaminobutanoic acid (DAB), ornithine (Orn), and isoserine (Ise). Of those, A, L, M, F, I, V, P, W, and G are classified as non-polar (hydrophobic) amino acids, and acidic amino acids, such as D and E, basic amino acids, such as R, K, H, DAP, DAB, and Orn, and polar non-charge amino acids, such as N, Q, S, T, C, Y, and Ise, are classified as polar (hydrophilic) amino acids.

### A. Peptide

A peptide according to an embodiment of the present invention includes a hydrophobic region, and a hydrophilic region positioned on at least one end side of the hydrophobic region and having hydrophobicity lower than that of the hydrophobic region, includes, at one end, a first target binding site capable of binding to a first target, and includes, at the other end, a second target binding site capable of binding to a second target. Peptides each having such configuration (hereinafter sometimes referred to as "self-associating peptides") can associate together through a hydrophobic interaction between the hydrophobic regions in an aqueous medium to form an assembly. As required, a drug may be bonded to the peptide. The drug may be grafted to, for example, a side chain of the peptide.

The number of amino acid residues for forming the self-associating peptide is, for example, from 10 to 40, preferably from 12 to 38, more preferably from 15 to 31, still more preferably from 18 to 24. The number of amino acid residues for forming the self-associating peptide means the number of amino acid residues for forming a main chain of the self-associating peptide, and the number of amino acid residues for forming each of the regions or sites described later also means the same.

The self-associating peptide may be formed of only natural amino acid residues, may be formed of only non-natural amino acid residues, or may contain both of natural amino acid residues and non-natural amino acid residues.

The self-associating peptide may be in the form of a derivative or a salt, as long as the effect of the present invention is obtained. As used herein, the peptide may be a derivative of the peptide, or a salt of the peptide or the derivative thereof, unless the derivative or the salt is inappropriate in the context.

Examples of the derivative of the peptide include derivatives obtained by substituting functional groups, such as an N-terminal amino group and a C-terminal carboxyl group of the peptide, and a carboxyl group, an amino group, a guanidino group, a hydroxyl group, and a thiol group on a side chain of the peptide, with various substituents. The substituent is not particularly limited, and examples thereof include an alkyl group, an acyl group, a hydroxy group, an amino group, an alkylamino group, a nitro group, an amide group, a sulfonyl group, a phosphoric acid group, a halogen, and various protective groups. Those substituents may each be further substituted with a halogen such as fluorine. In addition, the substitution may be performed by introducing a label, such as a fluorescent label or a biotin label.

The salt of the peptide is preferably a pharmacologically acceptable salt. Examples of the pharmacologically acceptable salt include an acid addition salt and a base addition salt. Examples of the acid addition salt include an inorganic acid salt and an organic acid salt. Examples of the inorganic acid salt include a hydrochloric acid salt, a hydrobromic acid salt, a sulfuric acid salt, a hydroiodic acid salt, a nitric acid salt, and a phosphoric acid salt. Examples of the organic acid salt include a citric acid salt, an oxalic acid salt, an acetic acid salt, a formic acid salt, a propionic acid salt, a benzoic acid salt, a trifluoroacetic acid salt, a maleic acid salt, a tartaric acid salt, a methanesulfonic acid salt, a benzenesulfonic acid salt, and a p-toluenesulfonic acid salt. Examples of the base addition salt include an inorganic base salt and an organic base salt. Examples of the inorganic base salt include a sodium salt, a potassium salt, a calcium salt, a magnesium salt, and an ammonium salt. Examples of the organic base salt include organic base salts, such as a triethylammonium salt, a triethanolammonium salt, a pyridinium salt, and a diisopropylammonium salt.

### A-1. First Embodiment

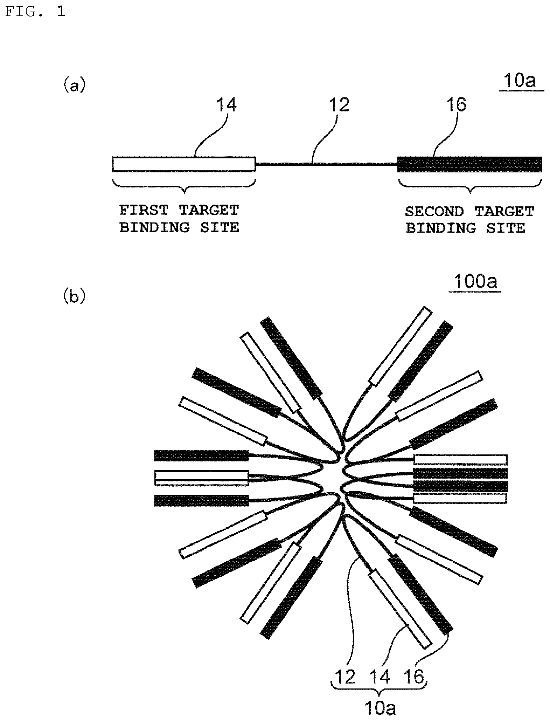

FIG. **1(a)** is a schematic view for illustrating a configuration of a self-associating peptide according to a first embodiment of the present invention. A self-associating peptide **10a** includes a hydrophobic region **12,** a first hydrophilic region **14** positioned on one end side of the hydrophobic region **12,** and a second hydrophilic region **16** positioned on the other end side. As illustrated in FIG. **1(b)****,** the self-associating peptides **10a** can associate together through a mutual hydrophobic interaction between the hydrophobic regions **12** in an aqueous medium to form a peptide assembly **100a.** In the self-associating peptide **10a,** any of a terminus on a first hydrophilic region **14** side and a terminus on a second hydrophilic region **16** side may be an N-terminus.

The hydrophobic region **12** typically has a non-polar amino acid residue or a tyrosine residue on each of its ends, and is formed of, for example, 2 to 15, preferably 3 to 13, more preferably 4 to 12, still more preferably 6 to 10 amino acid residues.

In one embodiment, for example, 60% or more, preferably 60% to 100%, more preferably 70% to 90% of the amino acid residues for forming the hydrophobic region are selected from a non-polar amino acid residue and a tyrosine residue. Tyrosine is classified as a polar amino acid, but has very low solubility and thus can contribute to the self-associating property of the peptide by being incorporated into the hydrophobic region.

In one embodiment, the total number of the non-polar amino acid residues and the tyrosine residues in the hydrophobic region is, for example, 2 or more, preferably from 3 to 13, more preferably from 4 to 10.

The non-polar amino acid residue in the hydrophobic region is selected from, for example, L, A, W, F, V, I, and G, preferably from L, A, W, F, and G, more preferably from L, A, W, and F. Those non-polar amino acid residues may be advantageous in terms of self-association of the peptide. The number of kinds of the non-polar amino acid residues in the hydrophobic region may be only one, or two or more.

The hydrophobic region may contain a polar amino acid residue. From the viewpoint of peptide synthesis, when the hydrophobic region contains only one kind of non-polar amino acid residue, the polar amino acid residues are preferably interspersed. The number of the polar amino acid residues that may be incorporated into the hydrophobic region is, for example, from 1 to 6, and may also be, for example, from 2 to 4. The ratio of the number of the polar amino acid residues to the number of all amino acid residues for forming the hydrophobic region is, for example, from 7% to 40%, and may also be, for example, from 13% to 30%. The number of kinds of the polar amino acid residues in the hydrophobic region may be only one, or two or more.

The use of the polar amino acid residue as the amino acid residue for forming the hydrophobic region allows flexible adjustment of hydrophobicity of the hydrophobic region in a wide range.

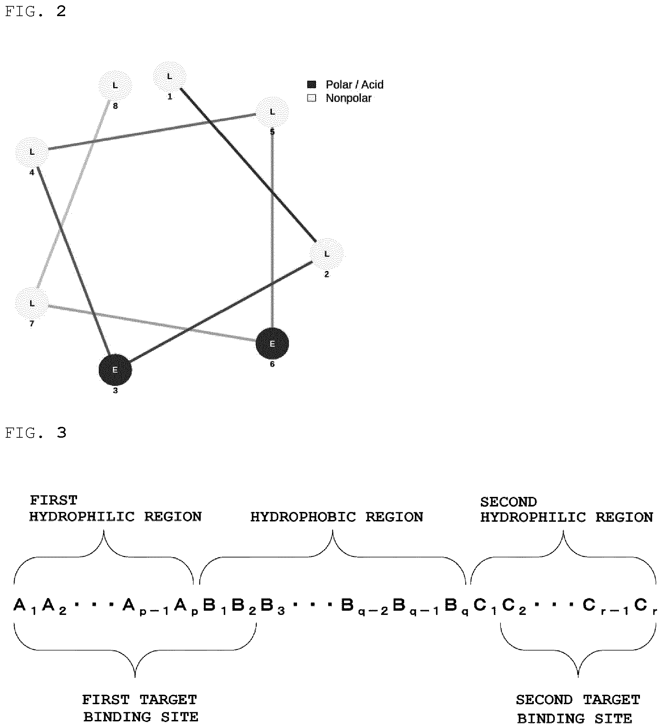

In one embodiment, the hydrophobic region contains a charged amino acid residue, preferably an acidic amino acid residue. The number of the charged amino acid residues in the hydrophobic region is, for example, from 1 to 6, or, for example, from 2 to 4. Two or more charged amino acid residues may be arranged continuously or non-continuously. For example, the charged amino acid residues may be arranged at regular intervals in the hydrophobic region. In one embodiment, the charged amino acid residues may be arranged in distinction from non-polar amino acid residues so as to gather on one side relative to a straight line passing through the central axis of an α-helix in a helical wheel projection, preferably so as to adjoin one another in a helical wheel projection as illustrated in FIG. **2****.** Such arrangement of the charged amino acid residues allows control of peptide aggregation. For example, a peptide in which polar amino acid residues such as charged amino acid residues are arranged so as to gather on one side of an α-helix structure can adopt a so-called amphiphilic helix structure, and can control the degree of aggregation by employing electrostatic repulsion caused by the structure. The helical wheel projection may be obtained by using amino acid sequence analysis software such as Gene Inspector^{™}.

The hydropathy index of the hydrophobic region is, for example, -1.6 or more, and may be preferably from -0.9 to 4.5, more preferably from 0.4 to 2.8. The term "hydropathy index of the hydrophobic region" as used herein means an average value of hydropathy indices of all amino acids for forming the hydrophobic region. Values described below are adopted as hydropathy indices of natural amino acids.

**Table 1**

| Amino acid | | Hydropathy index | Amino acid | | Hydropathy index |
|---|---|---|---|---|---|
| Arginine | R | -4.5 | Serine | S | -0.8 |
| Lysine | K | -3.9 | Threonine | T | -0.7 |
| Aspartic acid | D | -3.5 | Glycine | G | -0.4 |
| Asparagine | N | -3.5 | Alanine | A | 1.8 |
| Glutamic acid | E | -3.5 | Methionine | M | 1.9 |
| Glutamine | Q | -3.5 | Cysteine | C | 2.5 |
| Histidine | H | -3.2 | Phenylalanine | F | 2.8 |
| Proline | P | -1.6 | Leucine | L | 3.8 |
| Tyrosine | Y | -1.3 | Valine | V | 4.2 |
| Tryptophan | W | -0.9 | Isoleucine | I | 4.5 |

Each of the first hydrophilic region **14** and the second hydrophilic region **16** typically has a polar amino acid residue (except a tyrosine residue) at an end on a hydrophobic region **12** side and has hydrophobicity lower than that of the hydrophobic region **12.** A difference in hydrophobicity between the hydrophobic region and the first hydrophilic region or the second hydrophilic region is not limited as long as the self-associating peptides can associate together through a hydrophobic interaction between the hydrophobic regions in an aqueous medium to form an assembly. In the assembly, the hydrophobic region in the self-associating peptide is preferably arranged inside, and the first hydrophilic region and the second hydrophilic region are arranged outside. Differences between the hydropathy index of the hydrophobic region and the hydropathy index of the first hydrophilic region and between the hydropathy index of the hydrophobic region and the hydropathy index of the second hydrophilic region (hydropathy index of hydrophobic region-hydropathy index of hydrophilic region) are each independently, for example, 0 or more, and may be preferably from 0.4 to 6.4, more preferably from 1 to 6. The term "hydropathy index of the hydrophilic region" as used herein means an average value of hydropathy indices of all amino acids for forming the hydrophilic region.

The numbers of amino acid residues for forming the first hydrophilic region and the second hydrophilic region are each independently, for example, from 3 to 24, preferably from 3 to 18, more preferably from 3 to 15, still more preferably from 5 to 15.

The ratio of the number of amino acid residues for forming the first hydrophilic region to the number of amino acid residues for forming the hydrophobic region is not limited as long as the effect of the present invention is obtained, and the ratio is, for example, from 20% to 400%, preferably from 30% to 300%. The ratio of the number of amino acid residues for forming the second hydrophilic region to the number of amino acid residues for forming the hydrophobic region is not limited as long as the effect of the present invention is obtained, and the ratio is, for example, from 20% to 400%, preferably from 30% to 300%.

In one embodiment, for example, 20% or more, 30% or more, or 40% or more, preferably 50% to 100% of the amino acid residues for forming the first hydrophilic region or the second hydrophilic region are polar amino acid residues except a tyrosine residue. In addition, a difference between the ratio of the number of the polar amino acid residues except a tyrosine residue to the number of the amino acid residues for forming the hydrophilic region and the ratio of the number of the polar amino acid residues except a tyrosine residue to the number of the amino acid residues for forming the hydrophobic region is, for example, 10% or more, preferably 20% or more, and may be, for example, 30% or more, 40% or more, or 50% or more.

The self-associating peptides each containing a charged amino acid residue, that is, an acidic amino acid residue and/or a basic amino acid residue, in the hydrophilic region can form an assembly that may change in particle diameter through pH adjustment of an aqueous medium. In particular, the self-associating peptides each containing 1 or more, for example, 2 or more or 3 or more histidine residues in the hydrophilic region can form an assembly that may change in particle diameter in an approximately neutral to weakly-acidic range.

The self-associating peptide includes, at one end, a first target binding site capable of binding to a first target, and includes, at the other end, a second target binding site capable of binding to a second target. According to the self-associating peptide having such configuration, an assembly including the first target binding site and the second target binding site exposed outside can be formed, and an assembly capable of binding to a target of each target binding site can be obtained.

The first target binding site and the second target binding site include amino acid sequences that can selectively bind to the first target and the second target to form biological binding pairs with the targets, respectively. As such amino acid sequences, known amino acid sequences may be used, or amino acid sequences capable of selectively binding to targets of interest may be obtained by screening with use of mRNA display, cDNA display, phage display, or the like.

The first target and the second target may be appropriately selected in accordance with purposes. The first target and the second target are typically substances derived from a living body, viruses, and the like, and examples thereof may include substances specifically present in living cells such as cancer cells, bacteria, fungi, and viruses, or substances produced therefrom.

The first target and the second target may be different from each other, or may be the same. When the first target and the second target are different substances, a peptide having directivity to each of the two targets is obtained, and consequently, a DDS having directivity to each of the two targets can be achieved. Regardless of whether the first target and the second target are the same substance or different substances, when the peptide forms a self-assembly, its affinity (avidity) to a target per the peptide can be improved as compared to a single peptide that binds to the first target or the second target. When the first target and the second target are the same substance, the first target binding site and the second target binding site can bind to the same site or different sites of the substance. When the first target and the second target are the same substance, amino acid sequences of the first target binding site and the second target binding site may be the same as or different from each other.

In one embodiment, at least one of the first target binding site or the second target binding site has biological activity. According to such an embodiment, the peptide itself can have biological activity derived from an end region having biological activity and exhibit medical effect, and it is thus not always necessary for the peptide or the peptide assembly to carry a drug. The biological activity of the target binding site may be appropriately selected in accordance with, for example, applications of the peptide. The biological activity may be, for example, a hormonal action, a neurotransmission action, an antitumor action, an antimicrobial action, or a regulatory action of enzymatic activity.

The first target binding site and the second target binding site are positioned on both ends of the self-associating peptide, respectively. The numbers of amino acid residues for forming the sites are not particularly limited as long as the sites are capable of binding to the first target and the second target, and the numbers may each be, for example, from 3 to 24, and may each also be, for example, from 5 to 15. In the self-associating peptide illustrated in FIG. **1(a)****,** the first hydrophilic region **14** is identical to the first target binding site, and the second hydrophilic region **16** is identical to the second target binding site. However, it is not required that the hydrophilic region be identical to the target binding site.

For example, the self-associating peptide illustrated in FIG. **3** as an example includes a first hydrophilic region formed of "p" amino acid residues, a hydrophobic region formed of "q" amino acid residues, and a second hydrophilic region formed of "r" amino acid residues arranged in this order from the N-terminal side to the C-terminal side. The region from the amino acid residue (A₁) at the N-terminus to the second amino acid residue (B₂) from the N-terminal side of the hydrophobic region represents a first target binding site, and the region from the second amino acid residue (C₂) from the N-terminal side of the second hydrophilic region to the amino acid residue (Cᵣ) at the C-terminus represents a second target binding site.

Unlike the illustrated examples, the second target binding site may include part of the hydrophobic region beyond the second hydrophilic region, or the first target binding site may be part of the first hydrophilic region.

From the viewpoint of forming an assembly having a configuration in which both of the target binding sites are arranged outside, it is preferred that, for example, 3 to 15, preferably 6 to 10 amino acid residues be interposed between the C-terminal amino acid residue of the first target binding site and the N-terminal amino acid residue of the second target binding site.

### A-2. Second Embodiment

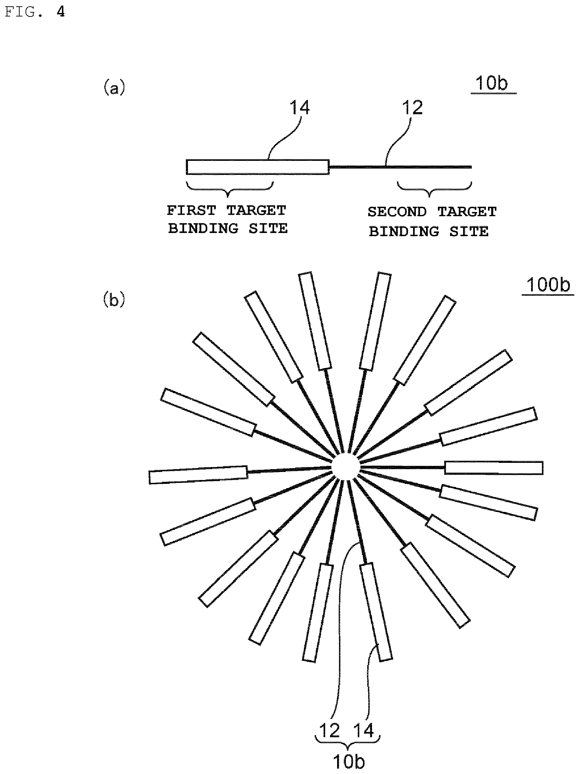

FIG. **4(a)** is a schematic view for illustrating a configuration of a self-associating peptide according to a second embodiment of the present invention. A self-associating peptide **10b** includes the hydrophobic region **12** and the first hydrophilic region **14** positioned on one end side of the hydrophobic region **12.** As illustrated in FIG. **4(b)****,** the self-associating peptides **10b** can associate together through a mutual hydrophobic interaction between the hydrophobic regions **12** in an aqueous medium to form a peptide assembly **100b.** In the self-associating peptide **10b,** any of a terminus on a hydrophobic region **12** side or a terminus on a first hydrophilic region **14** side may be an N-terminus.

The hydrophobic region **12** typically has a non-polar amino acid residue or a polar amino acid residue selected from a tyrosine residue, a serine residue, and a threonine residue at the terminus on the first hydrophilic region **14** side. The hydrophobic region preferably contains at least one kind selected from a proline residue, a tryptophan residue, a glycine residue, a tyrosine residue, a serine residue, and a threonine residue, and more preferably contains at least one kind selected from a tryptophan residue, a glycine residue, and a tyrosine residue. When a particle structure of the peptide assembly is excessively stable, the second target binding site, which is positioned at the terminus on the hydrophobic region **12** side, may fail to function sufficiently. However, such problem may be avoided by the hydrophobic region containing the above-mentioned amino acid residues because these amino acid residues each exhibit a negative value for a hydropathy index and have relatively low hydrophobicity. The hydrophobic region **12** is formed of, for example, 3 to 37, preferably 4 to 15, more preferably 5 to 13, still more preferably 6 to 12 amino acid residues.

In one embodiment, for example, 50% or more of the amino acid residues for forming the hydrophobic region are non-polar amino acid residues or polar amino acid residues selected from a tyrosine residue, a serine residue, and a threonine residue, and 50% or more (e.g., 50% to 80%) thereof are selected preferably from a proline residue, a tryptophan residue, a glycine residue, a tyrosine residue, a serine residue, and a threonine residue, more preferably from a tryptophan residue, a glycine residue, and a tyrosine residue.

In one embodiment, the total number of the non-polar amino acid residues and the polar amino acid residues selected from a tyrosine residue, a serine residue, and a threonine residue in the hydrophobic region is, for example, 3 or more, preferably from 4 to 12, more preferably from 6 to 10.

The non-polar amino acid residues for forming the hydrophobic region may vary depending on an amino acid sequence of a target binding site, but preferably contain the same non-polar amino acid residues as those described in the first embodiment in addition to the foregoing.

The hydrophobic region may further contain a polar amino acid residue except a tyrosine residue, a serine residue, and a threonine residue. The number of the polar amino acid residues (except a tyrosine residue, a serine residue, and a threonine residue) that may be incorporated into the hydrophobic region may be, for example, from 1 to 6, and may also be, for example, from 2 to 4. The ratio of the number of the polar amino acid residues (except a tyrosine residue, a serine residue, and a threonine residue) to the number of all amino acid residues for forming the hydrophobic region may be, for example, from 7% to 50%, and may also be, for example, from 13% to 30%. The number of kinds of the polar amino acid residues (except a tyrosine residue, a serine residue, and a threonine residue) in the hydrophobic region may be only one, or two or more. The number of charged amino acid residues in the hydrophobic region is, for example, from 0 to 6, or, for example, from 2 to 4.

The first hydrophilic region **14** typically contains a polar amino acid residue (except a tyrosine residue, a serine residue, and a threonine residue) at the terminus on the hydrophobic region **12** side, and has hydrophobicity lower than that of the hydrophobic region **12.** A difference in hydrophobicity between the hydrophobic region and the first hydrophilic region is not limited as long as the self-associating peptides associate together through a hydrophobic interaction between the hydrophobic regions in an aqueous medium to form an assembly. In the assembly, typically, the hydrophobic region of the self-associating peptide is arranged inside, and the first hydrophilic region is arranged outside. When an intermolecular hydrogen bond (cross-linking) can be formed in an aqueous solution, an aqueous peptide solution may exhibit a gel-like property. The hydropathy index of the hydrophobic region is as described for the first embodiment. A difference between the hydropathy index of the hydrophobic region and the hydropathy index of the first hydrophilic region (hydropathy index of hydrophobic region-hydropathy index of hydrophilic region) is, for example, -0.3 or more, and may be preferably from 0 to 6, more preferably from 0.4 to 5.

The number of amino acid residues for forming the first hydrophilic region is, for example, from 3 to 24, preferably from 5 to 15.

The ratio of the number of the amino acid residues for forming the first hydrophilic region to the number of the amino acid residues for forming the hydrophobic region is not limited as long as the effect of the present invention is obtained, and the ratio is, for example, from 25% to 400%, preferably from 50% to 200%.

In one embodiment, for example, 20% or more, 30% or more, or 40% or more, preferably 50% to 100% of the amino acid residues for forming the first hydrophilic region are polar amino acid residues. For example, 20% or more, 30% or more, 40% or more, or 50% or more of the amino acid residues for forming the first hydrophilic region may be polar amino acid residues except a tyrosine residue, a serine residue, and a threonine residue. In addition, a difference between the ratio of the number of the polar amino acid residues except a tyrosine residue, a serine residue, and a threonine residue to the number of the amino acid residues for forming the first hydrophilic region, and the ratio of the number of the polar amino acid residues except a tyrosine residue, a serine residue, and a threonine residue to the number of the amino acid residues for forming the hydrophobic region is, for example, 10% or more, preferably 20% or more, and may be, for example, 30% or more, 40% or more, or 50% or more.

The self-associating peptide **10b** of the illustrated example includes, at an end on the first hydrophilic region **14** side, a first target binding site capable of binding to a first target, and includes, at an end on the hydrophobic region **12** side, a second target binding site capable of binding to a second target. The self-associating peptides each having such configuration can form an assembly including the first target binding site exposed outside, and is capable of binding to the first target in the state of an assembly, and when an assembly structure collapses, the second target binding site can appear from the inside of the assembly and bind to the second target. Each of the first target binding site and the second target binding site is as described for the first embodiment.

### A-3. Production Method for Self-associating Peptide

Examples of a production method for the self-associating peptide include a chemical synthesis method, such as a solid-phase synthesis method, a stepwise extension method, or a liquid-phase synthesis method, a fermentation method, and an enzymatic method. Of those, a solid-phase synthesis method is preferred. Examples of the solid-phase synthesis method include a Fmoc synthesis method and a Boc synthesis method.

### B. Peptide Assembly

According to another aspect of the present invention, there is provided a peptide assembly including the peptide or the derivative thereof, or the salt thereof described in the section A. The peptide assembly may be formed by association of two or more molecules of the self-associating peptide through an intermolecular interaction. The intermolecular interaction for forming an assembly typically includes a hydrophobic interaction between hydrophobic regions of peptides, and may further include other intermolecular interactions, such as a hydrogen bond, an electrostatic interaction, or a van der Waals interaction between peptide molecules.

The peptide assembly is preferably a core-shell type assembly as illustrated in FIG. **1(b)** and FIG. **4(b)****.** In a core-shell type peptide assembly, the self-associating peptides associate together so as to arrange the hydrophobic regions inside (in a core) and the hydrophilic regions outside (in a shell). In addition, the self-associated peptides may associate together to form a larger assembly (e.g., a secondary assembly).

The average particle diameter of the peptide assembly may be any appropriate value in accordance with purposes. In intravenous administration, the average particle diameter of the peptide assembly is, for example, less than 300 nm, preferably 100 nm or less, more preferably from 5 nm to 50 nm. In use for another route of administration, the average particle diameter of the peptide assembly is, for example, 3,000 nm or less, and may be preferably 1,000 nm or less. The particle diameter of the peptide assembly may be adjusted by, for example, hydrophobicity of the hydrophobic region and/or the hydrophilic region, and a pH of an aqueous medium. Specifically, when the hydrophobic region and/or the hydrophilic region has higher hydrophobicity, the peptide assembly tends to have a smaller particle diameter. In addition, inclination of an equilibrium of electrolytic dissociation in a side chain of a charged amino acid residue from a dissociation state to a non-dissociation state leads to higher hydrophobicity of the hydrophobic region and/or the hydrophilic region, resulting in a tendency to provide the peptide assembly having a smaller particle diameter. In particular, when the content of histidine is high, the particle diameter of the peptide assembly can be adjusted in an approximately neutral to weekly-acidic range, which is to be a physiological condition.

In one embodiment, the self-associating peptide, which forms the peptide assembly, exhibits, as the whole peptide assembly, binding affinity to a first target equal to or more than that of a non-self-associating peptide including a first target binding site, and preferably exhibits higher binding affinity. Specifically, the dissociation constant (K_{D}) of the peptide assembly to the first target per peptide may be equal to or less than, preferably less than the dissociation constant (K_{D}) of the non-self-associating peptide including the first target binding site to the first target. The dissociation constant (K_{D}) of the non-self-associating peptide including the first target binding site to the first target is, for example, 10⁻⁶ M or less, and may be preferably 10⁻⁷ M or less.

In one embodiment, the self-associating peptide, which forms the peptide assembly, exhibits, as the whole peptide assembly, binding affinity to a second target equal to or more than that of a non-self-associating peptide including a second target binding site, and preferably exhibits higher binding affinity. Specifically, the dissociation constant (K_{D}) of the peptide assembly to the second target per peptide may be equal to or less than, preferably less than the dissociation constant (K_{D}) of the non-self-associating peptide including the second target binding site to the second target. The dissociation constant (K_{D}) of the non-self-associating peptide including the second target binding site to the second target is, for example, 10⁻⁶ M or less, and may be preferably 10⁻⁷ M or less. The dissociation constant (K_{D}) of the peptide assembly to the first target or the second target per peptide means binding affinity of the peptide assembly to the first target or the second target in terms of peptide, and may be obtained by calculating a dissociation constant through use of the molar concentration of a peptide used for forming the peptide assembly instead of the molar concentration of the assembly.

The peptide assembly may further include a drug. The peptide assembly can exhibit target directivity resulting from a target binding site. Thus, the peptide assembly including a drug can deliver the drug to a target of interest. The drug may be incorporated in the peptide assembly by being carried (e.g., encapsulated), or may be included in the peptide assembly by being bonded to the self-associating peptide (e.g., bonded to a side chain of the peptide, as required, via a linker).

Any appropriate drug may be used as the drug in accordance with purposes. Examples thereof include antitumor agents, signal transduction inhibitors, antimetabolites, analgesic agents, anti-inflammatory agents, antimicrobial agents, and contrast agents. Of those, hydrophobic drugs are preferred. The hydrophobic drug may be preferably carried inside the peptide assembly. Examples of hydrophobic antitumor agents include paclitaxel, topotecan, camptothecin, cisplatin, daunorubicine hydrochloride, methotrexate, mitomycin C, docetaxel, vincristine sulfate, and derivatives thereof.

The peptide assembly may be obtained by, for example: dissolving the self-associating peptide in an organic solvent; as required, drying up the resulting solution to form a film by air drying, for example, under a nitrogen stream atmosphere; further, as required, drying up the solution under reduced pressure to remove the organic solvent; then adding and mixing an aqueous medium to cause self-association of the self-associating peptides.

Examples of the organic solvent for dissolving the self-associating peptide include: non-water-miscible organic solvents, such as dichloromethane, chloroform, diethyl ether, dibutyl ether, ethyl acetate, and butyl acetate; water-miscible organic solvents, such as methanol, ethanol, propyl alcohol, isopropyl alcohol, dimethyl sulfoxide, dimethylformamide, dimethylacetamide, acetonitrile, acetone, and tetrahydrofuran; and mixed solvents thereof.

Examples of the aqueous medium may include water and a buffer. Examples of the buffer include a phosphate buffer, a phosphate buffered saline solution, a citrate buffer, a Tris buffer, a TAPS buffer, a MES buffer, and a HEPES buffer. The pH of the aqueous medium is, for example, from 3 to 8, preferably from 5 to 7.4.

Alternatively, the peptide assembly may be produced by stirring a mixture liquid containing the self-associating peptide and as required, a drug. The stirring of the mixture liquid is preferably performed by application of energy such as ultrasonic waves.

### C. Peptide Composition

According to another aspect of the present invention, there is provided a peptide composition including an aqueous medium and the peptide assembly described in the section B. As described in the section B, the peptide assembly is formed through association via an intermolecular interaction (typically, a hydrophobic interaction between the hydrophobic regions of the self-associating peptides). The peptide composition may further incorporate a drug. The drug may be carried (e.g., encapsulated) in the peptide assembly, or may be bonded to the self-associating peptide (e.g., bonded to a side chain of the peptide, as required, via a linker). The peptide assembly, the drug, and the aqueous medium are as described in the section B.

In one embodiment, the peptide composition is a pharmaceutical composition including a self-associating peptide having biological activity and/or a drug as an active ingredient, and in the pharmaceutical composition, the peptide assembly can function as a DDS for efficiently delivering the active ingredient to a target. In such an embodiment, the peptide composition is prepared into any appropriate dosage form in accordance with a mode of administration. For example, the peptide composition may be a parenteral preparation including an injection such as a subcutaneous injection, an intravenous injection, an intramuscular injection, an intraperitoneal injection, or an infusion; an eyedrop; a nasal drop; and a topical agent (e.g., an ophthalmic ointment and an oral ointment). Alternatively, the peptide composition may be an oral preparation in the form of preventing degradation with gastric acid, such as enteric coating.

The peptide composition may further include any appropriate additive in accordance with, for example, applications. Examples of the additive include a tonicity agent, a buffering agent, a preservative, a thickener, a stabilizer, and a pH adjuster.

### Examples

The present invention is specifically described below by way of Examples, but the present invention is not limited to these Examples.

### [Experimental Example 1: Synthesis of Peptide]

Peptides were synthesized by using a peptide synthesis service from Toray Research Center, Inc. Amino acid sequences of the peptides thus obtained are shown in Table 2.

In Table 2, the peptides of Examples are each separated into three regions by two hyphens. The N-terminal-side region and C-terminal-side region of each of the peptides of Examples 1-1 to 1-5 are a HA-binding site capable of binding to hydroxyapatite (HA) and an Rgp-binding site capable of binding to Arg-gingipain (Rgp), respectively. The N-terminal-side region and C-terminal-side region of each of the peptides of Examples 2-1 and 2-2 represent an amino acid sequence derived from a CD73-binding peptide capable of binding to CD73 and an amino acid sequence derived from an FcRn-binding peptide capable of binding to a neonatal Fc receptor (FcRn), respectively. The underlined area indicates a hydrophobic region. Meanwhile, the peptides of Reference Examples are amphiphilic peptides, and the N-terminal-side region and C-terminal-side region of each of the peptides separated by one hyphen are a hydrophilic region (also serving as an Rgp binding site) and a hydrophobic region, respectively. The amino acid sequences forming the respective target binding sites are the sequences described in previously reported literatures (e.g., P. Steinbauer, et al. Single-Molecule Force Spectroscopy Reveals Adhesion-by-Demand in Statherin at the Protein-Hydroxyapatite Interface Langmuir 2020, 36, 13292-13300.) or sequences determined by cDNA display.

**Table 2**

| | | Amino acid sequence | SEQ ID NO |
|---|---|---|---|
| Example | 1-1 | DSpSEEKW-LLELLELL-RRK(Tfa) RR | 1 |
| | 1-2 | DSpSEEKW-LLELLELL-RRKRR | 2 |
| | 1-3 | DSpSEEKW-WEWWEWW-RRK(Tfa) RR | 3 |
| | 1-4 | DSpSEEK-AAEAAEAA-RRK(Tfa) RR | 4 |
| | 1-5 | DSpSEEKW-LLELLELL-KGKILRPR | 5 |
| | 2-1 | GRSHHKRK-LLELLELL-HRHHVQER | 6 |
| | 2-2 | HHIRRKRQ-WWEWWEWW-HGDHHKSN | 7 |
| Reference Example | 1-1 | KGKILRPR-LLLLLLLL | 8 |
| | 1-2 | KGKILRPR-YYYYYYYY | 9 |
| | 1-3 | KGKILRPR-WWWWWWWW | 10 |
| | 1-4 | KGKILRPR-FFFFFFFF | 11 |
| | 1-5 | KGKILRPR-LLELLELL | 12 |
| | 1-6 | KGKILRPR-WWEWWEWW | 13 |
| | 1-7 | KGKILRPR-AAEAAEAA | 14 |
| | 1-8 | KGKILRPR-AAAAAAAA | 15 |

| | | | |
|---|---|---|---|
| *pS represents phosphorylated serine. *K(Tfa) represents lysine having a trifluoroacetylated amino group at the ε-position. *The C-terminal carboxyl group of each of the peptides except those of Examples 2-1 and 2-2 is amidated. | | | |

### [Experimental Example 2: Formation of Peptide Assembly]

5 mg of each of the peptides of Examples 1-1 to 1-5 and Reference Examples 1-1 to 1-8 was dissolved in dimethyl sulfoxide (DMSO) to provide a stock solution (40 mM). To a 10 mM HEPES and 150 mM NaCl buffer (pH 7) under stirring with a micromixer (E-36, Taitec Corporation), 4 µL of the stock solution was dropped to be diluted 100-fold. The dilution was vortexed and then left to stand still at room temperature overnight. Then, 200 µL of the dilution was used as a measurement sample and measurement of particle diameter distribution was performed at 25°C by dynamic light scattering with Litesizer 500 (Anton Paar GmBH). The results determined by defining the main peak of a scattering intensity as the average particle diameter of an assembly are shown in Table 3. Provided that, when a polydispersity index was more than 20%, the main peak was determined with further reference to a volume-based particle diameter distribution.

**Table 3**

| | | Average particle diameter (nm) |
|---|---|---|
| Example | 1-1 | 380 |
| | 1-2 | 250 |
| | 1-3 | 350 |
| | 1-4 | 450 |
| | 1-5 | 5 |
| Reference Example | 1-1 | 36 |
| | 1-2 | 210 |
| | 1-3 | 920 |
| | 1-4 | 50 |
| | 1-5 | 5 |
| | 1-6 | 10 |
| | 1-7 | 520 |
| | 1-8 | >1,000 |

As shown in Table 3, each of the peptides of Examples 1-1 to 1-5 formed an assembly having an average particle diameter of 500 nm or less in the HEPES buffer. In addition, each of the amphiphilic peptides of Reference Examples 1-1 to 1-8 was confirmed to form an assembly, and to have a particle diameter possibly changed depending on the amino acid sequence of the hydrophobic region. Example 1-5 and Reference Example 1-5 each provided an average particle diameter of 5 nm as measured, which was larger than the size of one peptide molecule, suggesting the formation of an assembly containing two or more peptide molecules.

### [Experimental Example 3: Evaluation of pH Dependency of Particle Diameter of Peptide Assembly]

5 mg of each of the peptides of Examples 2-1 and 2-2 was dissolved in DMSO to provide a stock solution (50 mg/mL). To a 10 mM MES and 150 mM NaCl buffer (pH 6.0) under stirring with a micromixer (E-36, Taitec Corporation), 4 µL of the stock solution was dropped to be diluted 100-fold. Thus, a dilution A was obtained. The dilution A was vortexed and then left to stand still at room temperature overnight. 100 µL of the dilution A was used and diluted with 300 µL of the same MES buffer or a 10 mM HEPES and 150 mM NaCl buffer (pH 7.4), and stirred for 1 hour. Thus, a dilution B was obtained. 200 µL of each of the dilution A and the dilution B after having been left to stand still overnight was used as a measurement sample and measurement of particle diameter distribution was performed at 25°C with Litesizer 500 (Anton Paar GmBH). The results of average particle diameters determined in accordance with Experimental Example 2 are shown in Table 4.

**Table 4**

| | | Average particle diameter (nm) | |
|---|---|---|---|
| | | pH 6 | pH 7 |
| Example | 2-1 | 440 | 4 |
| | 2-2 | 4,400 | 210 |

As shown in Table 4, the average particle diameter of an assembly of the peptide of Example 2-1 or 2-2 was smaller in the buffer at pH 7 than that in the buffer at pH 6, suggesting the pH dependency of the average particle diameter of such peptide assembly. This pH dependency of the average particle diameter is estimated to be based on, for example, a change in state of electrolytic dissociation of a side chain of a charged amino acid residue, particularly histidine, corresponding to the pH of an aqueous medium, resulting in a change in hydrophobicity of a hydrophilic region.

### [Experimental Example 4: Evaluation of Binding Affinity to Second Target (Rgp)]

The peptide of Example 1-2 and an Rgp-binding peptide A (RRKRR: SEQ ID NO: 16) forming the Rgp binding site of the peptide were each measured for its binding affinity to Rgp by SPR using "Biacore-T100" (GE Healthcare). The measurement was performed according to a common method. Specifically, the measurement of the peptide of Example 1-2 was performed through bonding streptavidin (Extravidin SIGMA E2511) to a chip (CM5 sensor chip) by amine coupling, and then immobilizing Rgp biotinylated with a 20-fold molar ratio of an NHS-biotinylation reagent (EZ-Link^{™} Sulfo-NHS-SS-Biotin, Thermo Fischer Scientific Inc.). Meanwhile, the measurement of the Rgp-binding peptide A was performed through directly immobilizing Rgp on a CM5 sensor chip by amine coupling from the viewpoint of measurement sensitivity.

Next, the peptide stock solution (in DMSO) was diluted with a running buffer (10 mM HEPES (pH 7.4), 150 mM NaCl, 0.05% Tween, 0.2% DMSO) to prepare peptide solutions having final peptide concentrations of 25 nM, 100 nM, 400 nM, and 1,600 nM. Those peptide solutions having the four concentrations were used to provide sensorgrams indicating weight changes caused by binding between Rgp immobilized on the chip and the peptide. The sensorgrams thus obtained were subjected to curve fitting to a theoretical formula (software "BIAevaluation", GE Healthcare), thereby calculating a dissociation constant K_{D}. The results are shown in Table 5.

**Table 5**

| | Sequence | Dissociation constant (K_{D}) |
|---|---|---|
| | | (M) |
| Example 1-2 | DSpSEEKW-LLELLELL-RRKRR | 1.3×10⁻⁹ |
| Rgp-binding peptide A | RRKRR | 4.0×10⁻⁷ |

As shown in Table 5, the self-associating peptide of Example 1-2 exhibited higher binding affinity to Rgp (second target) as compared to the Rgp-binding peptide A (non-self-associating peptide containing a second target binding site). This may be because the peptide of Example 1-2 forms an assembly in the peptide solution, thereby allowing binding to a target at multiple points and consequently generating an avidity effect.

### [Experimental Example 5: Evaluation of Binding Affinity to First Target (HA)]

The peptides of Examples 1-2 and 1-5 and a HA-binding peptide A (DSpSEEKW: SEQ ID NO: 17) forming the HA binding site of each of the peptides were each evaluated for peptide binding affinity to hydroxyapatite beads (1 µm BcMag^{™} Hydroxyapatite Magnetic Particles (Bioclone Inc., catalog No. FO-101)). Specifically, the beads were weighed and suspended in a 100 mM sodium phosphate buffer (pH 7.4)/20% ethanol so as to have 30 mg/mL. 267 µL of the suspension of the beads ultrasonicated to be dispersed homogenously was dispensed in a 1.5 mL tube. The beads were fixed with a magnetic stand (Takara Bio Inc., Magnetic Stand (6 tubes), product code: 5328), followed by removal of a supernatant, and then 100 µL of a peptide solution was added. The peptide solution was prepared by diluting the 100 mM stock solution (in DMSO) to 40 µM with 10 mM HEPES (pH 7.4)/1% DMSO and further serially diluting the solution from 4-fold to 5-fold. After stirring at 4°C for 4 hours, the tube was stood in the magnetic stand for fixing the beads, and a supernatant was recovered. A peptide concentration in the supernatant was measured with a plate reader (VARIO-SCAN, Thermo Fischer Scientific Inc.) based on a fluorescence intensity derived from tryptophan in a sequence (excitation wavelength: 280 nm; emission wavelength: 355 nm) through use of 50 µL of the supernatant. The amount of the peptide bound to the beads was determined by subtracting the peptide concentration in the supernatant from a total peptide concentration, and the adsorption isotherm thus obtained was subjected to curve fitting to a theoretical formula (Microsoft Excel, Microsoft Corporation), thereby determining a dissociation constant K_{D} of the peptide to the hydroxyapatite beads. The results are shown in Table 6.

**Table 6**

| | Sequence | Dissociation constant (K_{D}) |
|---|---|---|
| | | (M) |
| Example 1-2 | DSpSEEKW-LLELLELL-RRKRR | 1.4×10⁻⁶ |
| Example 1-5 | DSpSEEKW-LLELLELL-KGKILRPR | <4.0×10⁻⁸ |
| HA-binding peptide A | DSpSEEKW | 7.1×10⁻⁷ |

As shown in Table 6, the self-associating peptide of Example 1-2 exhibited binding affinity to HA (first target) approximately comparable to that of the HA-binding peptide A (non-self-associating peptide containing a first target binding site). The results of Experimental Examples 4 and 5 demonstrated that the self-associating peptide of Example 1-2 was capable of binding to both the first target and the second target with affinity approximately comparable to or higher than that of each target-binding peptide alone. Meanwhile, the self-associating peptide of Example 1-5 exhibited binding affinity to HA higher than that of the HA-binding peptide A. Those results show that a second hydrophilic region (corresponding to a second target binding site in each of those peptides) may influence a secondary structure of a self-associating peptide as a whole, a manner of assembly formation, and a particle diameter, and consequently also influence affinity to the first target of the first target binding site. A difference between the results of Examples 1-2 and 1-5 may be attributed to a difference in likelihood of generating an avidity effect, which allows binding to a target at multiple points.

### Experimental Example 6: Formation of Pyrene-encapsulated Assembly

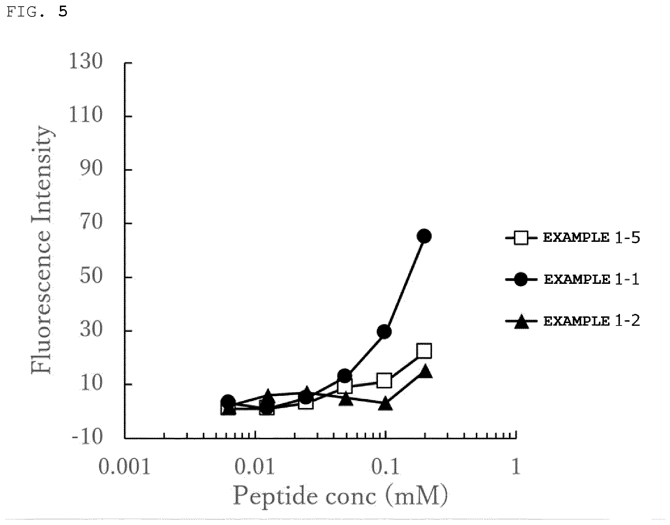

Fluorescence probing has hitherto been widely used as a technique for measuring the critical micellar concentration (CMC) of a surfactant solution. When hydrophobic fluorescent molecules are added to an aqueous solution of a surfactant and excited, week fluorescence is generated from the fluorescent molecules dispersed in the solution under a state in which the concentration of the surfactant is low. Meanwhile, the concentration of the surfactant is more than the CMC, the hydrophobic fluorescent molecule is incorporated into a micelle surrounded by hydrophobic groups of the surfactant (hydrophobic environment) and emits strong fluorescence. In this experimental example, a change in fluorescent property with a change in peptide concentration was investigated by using pyrene as a fluorescence probe and using a peptide instead of the surfactant.
(1) Pyrene (FUJIFILM Wako Pure Chemical Corporation) was weighed and dissolved with a vortex mixer by addition of 80% ethanol to prepare a 5 mM stock solution. The stock solution was diluted 500-fold with 80% ethanol to be adjusted to 10 µM. Meanwhile, for the peptide, the DMSO stock solution was diluted with PBS (BupH^{™} Phosphate Buffered Saline Packs, Thermo Fischer Scientific Inc.) to prepare a series of doubling dilution of tenfold of the final concentration (2 mM, 1 mM, 0.5 mM, 0.25 mM, 0.125 mM, and 0.0625 mM).
(2) To a 1.5 mL tube were added 142.4 µL of PBS, 16 µL of the peptide solution (final concentration: 0.2 mM, 0.1 mM, 0.05 mM, 0.025 mM, 0.0125 mM, or 0.00625 mM), and 1.6 µL of the 10 µM pyrene solution (final concentration: 0.1 µM), and mixed with a vortex mixer. As a control, 158.4 µL of PBS and 1.6 µL of the 10 µM pyrene solution were added to a 1.5 mL tube and mixed with a vortex mixer. Then, the mixtures were light-shielded and left to stand still at room temperature for 24 hours.
(3) One hundred fifty microliter of the reaction solution was transferred to each well of a plate (Non-Treated Black Polystyrene 96-well plate (Corning 3694)). As a control, 150 µL of PBS was added to a well. A fluorescent spectrum was scanned with a plate reader (SPARK^{™} multi-detection mode microplate reader, Tecan Group Ltd.) (excitation wavelength: 333 nm; emission wavelength: 360 nm to 420 nm). The peak value of a fluorescence intensity (384 nm) was plotted against a peptide concentration (logarithmic value). The results are shown in FIG. **5****.**

In the peptide of Example 1-1, the fluorescence intensity clearly increased as the peptide concentration increased. This increased fluorescence intensity means formation of a peptide assembly that provides a hydrophobic environment capable of encapsulating pyrene. In addition, in the plot diagram of FIG. **5****,** when a concentration at the intersection (inflection point) of two straight lines to be obtained was defined as a critical aggregation concentration (CAC), the CAC value of the peptide of Example 1-1 was 50 µM. The peptides of Examples 1-2 and 1-5 each had approximately the same fluorescence intensity up to 0.1 mM. Although any of the peptides exhibited a tendency to increase in fluorescence intensity at 0.2 mM, determination of their CAC values was judged as difficult.

### [Experimental Example 7: Formation of ANS-encapsulated Assembly]

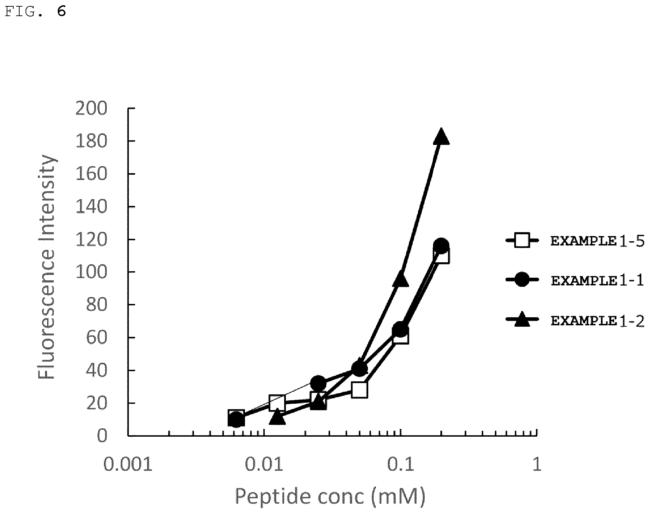

(1) 8-Anilino-1-naphthalenesulfonic acid (ANS) (Tokyo Chemical Industry Co., Ltd.) was weighed and dissolved by addition of 80% ethanol to prepare a 5 mM stock solution. The stock solution was diluted 500-fold with 80% ethanol to be adjusted to 10 µM. Meanwhile, for the peptide, a series of doubling dilution of solutions (2 mM, 1 mM, 0.5 mM, 0.25 mM, 0.125 mM, and 0.0625 mM) was prepared in the same manner as in Experimental Example 6.
(2) To a 1.5 mL tube were added 142.4 µL of PBS, 16 µL of the peptide solution (final concentration: 0.2 mM, 0.1 mM, 0.05 mM, 0.025 mM, 0.0125 mM, or 0.00625 mM), and 1.6 µL of the 10 µM ANS solution (final concentration: 0.1 µM), and mixed with a vortex mixer. As a control, 158.4 µL of PBS and 1.6 µL of the 10 µM ANS solution were added to a 1.5 mL tube and mixed. Then, the mixtures were light-shielded and left to stand still at room temperature for 1 hour.
(3) One hundred fifty microliter of the reaction solution was transferred to each well of a plate (Non-Treated Black Polystyrene 96-well plate (Corning 3694)). As a control, 150 µL of PBS was added to a well. A fluorescent spectrum was scanned with a plate reader (SPARK^{™} multi-detection mode microplate reader, Tecan Group Ltd.) (excitation wavelength: 370 nm; emission wavelength: 450 nm to 600 nm). A fluorescence intensity at 490 nm was plotted against a peptide concentration (logarithmic value). The results are shown in FIG. **6****.**

As shown in FIG. **6****,** any of the peptides increased in fluorescence intensity as the peptide concentration increased. ANS notably increases in fluorescence intensity under an hydrophobic environment as compared to under pure water, and thus has been widely used as a probe molecule like pyrene. This increased fluorescence intensity means formation of a peptide assembly that provides a hydrophobic environment capable of encapsulating ANS. Nevertheless, in consideration of the results of pyrene shown in FIG. **5** and the fact that pyrene is more hydrophobic than ANS, it can be estimated that hydrophobic environments capable of being presented by the peptide assemblies of Examples 1-2 and 1-5 at, for example, 0.1 mM can encapsulate ANS, but does not serve as an environment having high hydrophobicity enough to encapsulate pyrene. In addition, in the plot diagram of FIG. **6****,** when a concentration at the intersection (inflection point) of two straight lines to be obtained was defined as a CAC, the CAC value of any of the peptides was 50 µM.

Through Experimental Examples 6 and 7, formation of a peptide assembly was confirmed by not only measurement of particle diameters by dynamic light scattering but also fluorescence probing. In addition, it is revealed that an assembly-forming ability and a hydrophobic environment capable of being presented can change depending on a peptide sequence.

### [Experimental Example 8: Evaluation of Blood Kinetics]

Three kinds of fluorescein (FAM)-labeled peptides shown in Table 7 were purchased from Eurofins Genomics K.K. 1 mg of each of the peptides was separately dissolved in 4 mL of PBS to prepare an administration solution (0.25 mg/mL).

In each of the peptides, "GYSEWRKWEL" is a peptide sequence confirmed to have affinity to IL-6R, "HWRGWV" is a peptide sequence reported to have affinity to the Fc region of immunoglobulin (e.g., Journal of Chromatography A, 1218, (2011), 8344-8352), and each of the sequences can function as a target binding site. The peptide of Example 3-1 has: a hydrophobic region (LLLLLLLGG), which is indicated by the underline in Table 7; and a first hydrophilic region (GYSEWRKWE) and a second hydrophilic region (HWRGWV), which are positioned on the N-terminal side and C-terminal side of the peptide, and both of which have hydrophobicity lower than that of the hydrophobic region. The peptides of Example 3-1 and Comparative Example 2 have been confirmed to form an assembly in water.

**Table 7**

| | Amino acid sequence (all the C-termini are amidated) | Number of residues | SEQ ID NO |
|---|---|---|---|
| Comparative Example 1 | 5 (6)-FAM-GYSEWRKWEL | 10 | 18 |
| Comparative Example 2 | 5 (6)-FAM-GYSEWRKWEL-LLLLLL | 16 | 19 |
| Example 3-1 | 5 (6)-FAM-GYSEWRKWEL-LLLLLLGG-HWRGWV | 24 | 20 |

Eighteen Crl:CD1 (ICR) mice (male) (Jackson Laboratory Japan, Inc.) were subjected to 7 or more days of adaptation after their arrival and receipt, then divided by three per group into the six groups as in Table 8, and used in the following experiments. The administration solution (1 mg/4 mL/kg) was administered to the tail vein of a non-fasting mouse through use of a syringe equipped with an injection needle. A dose for each individual was calculated from a body weight measured on the day of the administration.

After the completion of the administration, about 0.1 mL of blood was collected from a jugular vein without anesthesia at the predetermined time points of collection shown in Table 8 through use of a heparin-treated syringe equipped with an injection needle. At the final time point of collection, whole blood was collected from a posterior aorta under isoflurane anesthesia. The collected blood was promptly transferred to a polypropylene tube and cooled on ice. The collected whole blood was centrifuged (about 10,000×g, 3 minutes, 4°C), and the plasma sample was promptly dispensed and light-shielded in a sample storage container. The plasma samples were cryopreserved until measurement (control temperature: about -80°C).

**Table 8**

| Group | Number of cases | Administration solution | Time point of collection |
|---|---|---|---|
| 1 | 3 | Comparative Example 1 | 0.05 hour, 1 hour, and 6 hours |
| 2 | 3 | Comparative Example 1 | 0.5 hour, 3 hours, and 12 hours |
| 3 | 3 | Comparative Example 2 | 0.05 hour, 3 hours, and 12 hours |
| 4 | 3 | Comparative Example 2 | 1 hour, 6 hours, and 24 hours |
| 5 | 3 | Example 3-1 | 0.05 hour, 3 hours, and 24 hours |
| 6 | 3 | Example 3-1 | 1 hour, 6 hours, and 48 hours |

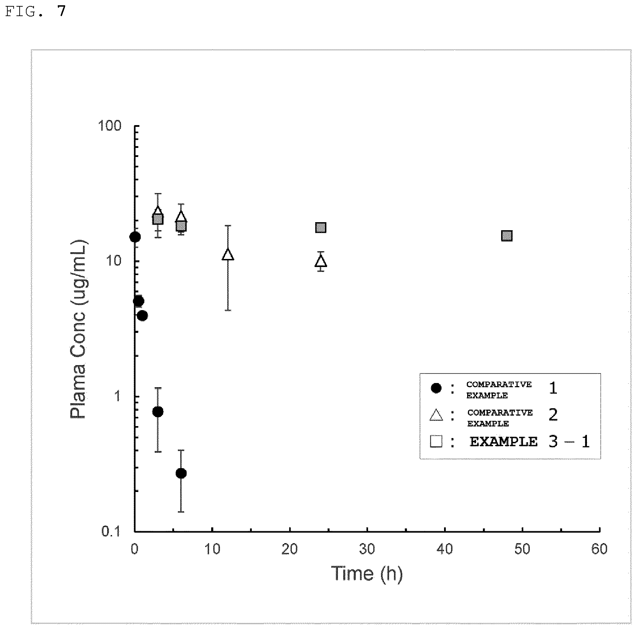

A freeze-thawed plasma sample (10 uL) was diluted 10-fold with PBS (90 µL), and measured for its fluorescence intensity with a plate reader (SPARK^{™} multi-detection mode microplate reader, Tecan Group Ltd.) (excitation wavelength: 495 nm; emission wavelength: 520 nm). The peptide concentration in plasma was calculated from a calibration curve created with each of Comparative Example 1, Comparative Example 2, and Example 3-1. The peptide concentrations in plasma thus obtained were logarithmically plotted against post-administration time, four time points including the final blood collection point were exponentially approximated (Microsoft Excel, Microsoft Corporation), and an elimination half-life was calculated from a slope of the straight line. The results are shown in FIG. 7 and Table 9. In FIG. 7, a symbol indicates a mean value, and a bar indicates standard deviation, and, when absent, is included in the symbol (n=3).

**Table 9**

| | Amino acid sequence (all the C-termini are amidated) | Elimination half-life (h) |
|---|---|---|
| Comparative Example 1 | 5 (6)-FAM-GYSEWRKWEL | 1.3 |
| Comparative Example 2 | 5 (6)-FAM-GYSEWRKWEL-LLLLLL | 16.7 |
| Example 3-1 | 5 (6)-FAM-GYSEWRKWEL-LLLLLLGG-HWRGWV | 133 |

As is clear from FIG. 7 and Table 9, the peptide of Example 3-1 has a remarkably extended half-life after intravenous administration as compared to the peptides of Comparative Example 1 and Comparative Example 2. In addition, in comparison between Comparative Example 1 and Comparative Example 2, Comparative Example 2 exhibited a longer half-life. Such improving effect on blood retention is conceived to be caused by an increase in apparent molecular weight of the peptide through formation of an assembly. Further, in Example 3-1, it conceived that the half-life remarkably longer than that of Comparative Example 2 is exhibited because of the influence of, for example, the function of the peptide sequence HWRGWV to interact with the Fc region of immunoglobulin present in blood.

### Industrial Applicability

The peptide, the peptide assembly, or the peptide composition according to the embodiment of the present invention can be preferably used in, for example, a DDS system and a pharmaceutical composition.

### Reference Signs List

- **10**: self-associating peptide

- **12**: hydrophobic region
- **14**: first hydrophilic region
- **16**: second hydrophilic region
- **100**: peptide assembly

## Claims

1. A peptide or a derivative thereof, or a salt thereof,
comprising a hydrophobic region, and a hydrophilic region positioned on at least one end side of the hydrophobic region and having hydrophobicity lower than that of the hydrophobic region,
comprising, at one end, a first target binding site capable of binding to a first target, and
comprising, at the other end, a second target binding site capable of binding to a second target.

2. The peptide or the derivative thereof, or the salt thereof according to claim 1, wherein the first target and the second target are different from each other.

3. The peptide or the derivative thereof, or the salt thereof according to claim 1, wherein the number of amino acid residues for forming the peptide or the derivative thereof, or the salt thereof is from 10 to 40.

4. The peptide or the derivative thereof, or the salt thereof according to claim 1, wherein the peptide or the derivative thereof, or the salt thereof comprises the hydrophilic regions on both end sides of the hydrophobic region.

5. The peptide or the derivative thereof, or the salt thereof according to claim 4, wherein the number of amino acid residues for forming the hydrophobic region is from 2 to 15, and 60% or more of the amino acid residues are selected from a nonpolar amino acid residue and a tyrosine residue.

6. The peptide or the derivative thereof, or the salt thereof according to claim 4, wherein the numbers of amino acid residues for forming the two hydrophilic regions are each independently from 3 to 24.

7. The peptide or the derivative thereof, or the salt thereof according to claim 1, wherein the peptide or the derivative thereof, or the salt thereof comprises the hydrophilic region on only one end side of the hydrophobic region.

8. The peptide or the derivative thereof, or the salt thereof according to claim 1, wherein at least one of the first target binding site or the second target binding site has biological activity.

9. A peptide assembly, comprising the peptide or the derivative thereof, or the salt thereof of any one of claims 1 to 8.

10. The peptide assembly according to claim 9, further comprising a drug.

11. The peptide assembly according to claim 9, wherein the peptide assembly satisfies at least one of the following item (i) or (ii):
(i) binding affinity of the peptide assembly to the first target per the peptide or the derivative thereof, or the salt thereof is equal to or more than binding affinity of a non-self-associating peptide including the first target binding site to the first target; and
(ii) binding affinity of the peptide assembly to the second target per the peptide or the derivative thereof, or the salt thereof is equal to or more than binding affinity of a non-self-associating peptide including the second target binding site to the second target.

12. A peptide composition, comprising an aqueous medium and the peptide assembly of claim 9,
wherein the peptide assembly is formed through association via an intermolecular interaction including a hydrophobic interaction between the hydrophobic regions of the peptides.

13. The peptide composition according to claim 12, wherein the peptide assembly further comprises a drug.
